## Europäisches Patentamt
## European Patent Office
## Office européen des brevets

(11) Veröffentlichungsnummer: **0 339 313**
**A1**

## (12) EUROPÄISCHE PATENTANMELDUNG

(21) Anmeldenummer: **89105940.4**

(22) Anmeldetag: **05.04.89**

(51) Int. Cl.⁴: **A61N 1/08 , A61N 1/18**

(30) Priorität: **19.04.88 DE 3813013**

(43) Veröffentlichungstag der Anmeldung:
**02.11.89 Patentblatt 89/44**

(84) Benannte Vertragsstaaten:
**AT BE CH DE ES FR GB IT LI LU NL SE**

(71) Anmelder: **HENNEBERG & BRUNNER Entwicklungsbüro, Goldbachstrasse 3 D-6991 Igersheim(DE)**

Anmelder: **CARLE GMBH + CO. KG medizinische Geräte, Einrichtungen und Präparate, Beim Braunstall 4 D-6990 Bad Mergentheim(DE)**

(72) Erfinder: **Henneberg, Fred Goldbachstrasse 3 D-6991 Igersheim(DE)**
Erfinder: **Brunner, Elmar Goldbachstrasse 3 D-6991 Igersheim(DE)**

(74) Vertreter: **Tergau, Enno et al Tergau & Pohl Patentanwälte Hefnersplatz 3 Postfach 119347 D-8500 Nürnberg 11(DE)**

(54) **Reizstromgerät.**

(57) Die Erfindung betrifft ein Reizstromgerät zur Elektrotherapie und/oder medizinisch-diagnostischen Anwendung unter Applikation eines Patientenstromes vorwählbarer Kurvenform und einstellbarer Maximalamplitude. Ein Funktionsgenerator erzeugt Spannungsimpulse vorwählbarer Kurvenform. Über eine regelbare Gleichspannungsquelle wird die gewünschte Reizstrom-Maximalamplitude eingestellt. Dem Funktionsgenerator und der Gleichspannungsquelle ist eine Ansteuerschaltung nachgeschaltet, die den Patientenstromkreis (4) entsprechend der vorgewählten Kurvenform und der eingestellten Reizstrom Maximalamplitude steuert. Eine Überwachungsschaltung überwacht die zwischen den Applikationselektroden (5,6) des Patientenstromkreises (4) auftretende Impedanz. Die regelbare Gleichspannungsquelle ist als aufund entladbarer Integrator (7) ausgebildet, der in seiner Spannung mittels eines zwischen ihm und dem Patientenanschluß (Elektroden 5,6) des Patientenstromkreises (4) eingeschalteten Impedanzerfassungsschaltung (8) auf den Bereich regelbarer Stromkonstanz begrenzbar ist.

## Reizstromgerät

Die Erfindung betrifft ein Reizstromgerät mit den im Oberbegriff des Anspruches 1 angegebenen Merkmalen.

Bei der Elektrotherapie bzw. elektromedizinischen Diagnostik wird mittels eines Reizstromgerätes über Applikationselektroden ein Strom vorwählbarer Kurvenform und einstellbarer Maximalamplitude durch entsprechende Körperteile eines Patienten geleitet. Ein Reizstromgerät weist dafür einen Funktionsgenerator zur Erzeugung von Spannungsimpulsen der vorwählbaren Kurvenform auf. Eine regelbare Gleichspannungsquelle dient zur Einstellung der Reizstrom-Maximalamplitude. Dem Funktionsgenerator und der Gleichspannungsquelle ist eine Ansteuerschaltung nachgeschaltet, die zur Steuerung des Patientenstromkreises entsprechend der vorgewählten Kurvenform und eingestellten Reizstrom-Maximalamplitude dient. Diese Ansteuerschaltung besteht beispielsweise aus einem Multiplizierer und einem dessen Ausgang nachgeschalteten Leistungstransistor als Endstufe zur Steuerung des Patientenstromkreises. Die beiden Eingänge des Multiplizierers werden mit den Spannungsimpulsen des Funktionsgenerators bzw. der Ausgangsspannung der regelbaren Gleichspannungsquelle gespeist.

Eine Überwachungsschaltung kontrolliert die zwischen den Applikationselektroden des Patientenstromkreises auftretende Impedanz. Eine derartige Schaltung ist beispielsweise aus DE-PS 22 57 189 bekannt. Nach dieser Druckschrift ist die Überwachungsschaltung in Form einer parallel zum Patientenwiderstand geschalte ten Überspannungsanzeige ausgebildet, die bei Auftreten einer Spannung von einigen Volt unter der Versorgungsspannung des Patientenstromkreises ein optisches Signal abgibt und damit anzeigt, daß die ordnungsgemäße Funktion nicht mehr gewährleistet ist.

Beim Einsatz von Reizstromgeräten sind nun verschiedene Besonderheiten zu beachten. So erfolgt die Messung der Reizstrom-Maximalamplitude in der Regel nicht durch direkt in den Patientenstromkreis eingeschaltete Spitzenstrommeßgeräte, sondern beispielsweise durch eine Messung der Gleichspannung der regelbaren Gleichspannungsquelle bei den Reizstromgeräten der eingangs genannten Art bzw. durch Ablesen der Potentiometerstellung bei den Reizstromgeräten nach DE-PS 22 57 189.

Tritt nun zwischen den Applikationselektroden eine hohe Impedanz auf - beispielsweise wegen eines unnatürlich hohen Patientenwiderstandes bzw. wegen hoher Übergangswiderstände zwischen den Applikationselektroden und dem Patienten selbst -. so kann die Versorgungsspannung des Patientenstromkreises nicht mehr ausreichen, die gewünschte Maximalamplitude des Reizstromes zu erzeugen. Die tatsächliche Maximalamplitude weicht also erheblich von der gewünschten und an sich am Gerät ablesbaren Maximalamplitude ab. Weiterhin ist es besonders gefährlich, wenn während der Behandlung eine oder beide Applikationselektroden vom Patienten abfallen, wie es in der Praxis wegen unzureichend angebrachter Elektroden oft passieren kann. Dann liegt zwischen den Elektroden eine der gewünschten Reizstrom-Maximalamplitude entsprechende Spannung an den offenen Elektroden an. Werden diese ohne vorherige Abschaltung der Elektroden-Versorgungsspannung wieder in Kontakt mit dem Patienten gebracht, so erleidet dieser einen Stromschlag, der äußerst unangenehm sein und zu starken Muskelzuckungen oder Hautreizungen führen kann. Die bei dem Reizstromgerät nach DE-PS 22 57 189 angegebene Überwachungseinrichtung zeigt zwar solche Störfälle an, jedoch sind keine Maßnahmen angegeben, mittels denen die vorgenannten Sicherheits- und Funktionsnachteile vermieden werden könnten.

Der Erfindung liegt davon ausgehend die Aufgabe zugrunde, ein Reizstromgerät der eingangs genannten Art sicherheitstechnisch und in funktionaler Hinsicht zu verbessern.

Die Lösung dieser Aufgabe ist in den kennzeichnenden Merkmalen des Anspruches 1 angegeben. Demnach ist die regelbare Gleichspannungsquelle als auf- und entladbarer Integrator ausgebildet, dessen Spannung proportional ist zur gewünschten Reizstrom-Maximalamplitude. Durch eine zwischen ihm und dem Patientenanschluß des Patientenstromkreises eingeschaltete Impedanzerfassungsschaltung ist der Integrator in seiner Spannung auf den Bereich regelbarer Stromkonstanz begrenzbar. Dies bedeutet, daß bei einer unverhältnismäßig hohen Impedanz zwischen den Applikationselektroden - beispielsweise aufgrund eines unnatürlich hohen Patientenwiderstandes bzw. wegen hoher Übergangswiderstände zwischen den Applikationselektroden und dem Patienten - die Integratorspannung abgesenkt wird, bis diese sich im Bereich regelbarer Stromkonstanz befindet. Damit ist die gesamte Schaltung nicht mehr übersteuerbar und die als Meßbasis zur Anzeige der Reizstrom-Maximalamplitude dienende Spannung am Integrator entspricht immer der tatsächlich auftretenden Reizstrom-Maximalamplitude.

Weiterhin wird bei einem Elektrodenabfall eine unendlich hohe Impedanz zwischen den Applikationselektroden geschaffen, die durch die Impedanzerfassungsschaltung mit der Folge detektierbar ist, daß die Integratorspannung mit einer durch die äußere Beschaltung gegebenen Zeitkonstante auf einen Minimalwert heruntergefahren wird. Dies wirkt über die Ansteuerschaltung derart zurück, daß zwischen den Applikationselektroden keine oder nur eine unschädlich geringe Spannung anliegt. Folglich können die Applikationselektroden ohne Gefahr für den Patienten wieder angelegt werden und durch ein entsprechendes Hochregeln der Gleichspannungsquelle die vorgesehene Reizstrom-Maximalamplitude von neuem eingestellt werden Durch die erfindungsgemäße Impedanzerfassungsschaltung wird also auf einfache Weise eine erhebliche Verbesserung der Sicherheit von Reizstromgeräten erzielt, wie sie von der einschlägigen VDE-Vorschriften und der medizinischen Geräteverordnung für medizinische elektrische Geräte gefordert wird.

Ein weiterer Vorteil des Erfindungsgegenstandes kommt bei der monopolaren Muskelreizung zum Tragen. Bei diesem Therapieverfahren wird jeweils während einer bestimmten Zeitdauer eine Einzelelektrode auf verschiedene Körperpartien aufgesetzt. Beim Versetzen der Elektrode wird der Patientenstromkreis kurzzeitig unterbrochen. Bei dieser kurzzeitigen Unterbrechung erfolgt zwar eine Entladung des Integrators mit seiner Zeitkonstante, die Spannung der Gleichspannungsquelle fällt jedoch nicht sofort auf Null ab. Es ist also eine quasi unterbrechungslose Behandlung möglich, nur ab und zu muß die der gewünschten Reizstrom-Maximalamplitude entsprechende Spannung der Gleichspannungsquelle durch Betätigung deren entsprechenden Einstellgliedes wieder auf ihren gewünschten Wert gesetzt werden.

In den Unteransprüchen sind vorteilhafte Ausgestaltungen des Erfindungsgegenstandes angegeben. So kennzeichnen die Ansprüche 2 und 3 eine einfache, jedoch wirkungsvolle Ausgestaltung der Impedanzerfassungsschaltung. Über einen Triggertransistor wird ein Steuersignal entsprechend der jeweils zwischen den Applikationselektroden herrschenden Impedanz erzeugt. Dieses Steuersignal dient als Schaltsignal für einen weiteren Transistor, über dessen Kollektor-Emitterstrecke der Integrator zur Spannungsbegrenzung auf den Bereich regelbarer Stromkonstanz entladbar ist. Das Steuersignal kann natürlich zur Ansteuerung weiterer nachgeschalteter Erfassungs-, Überwachungs- oder Anzeigevorrichtungen dienen.

Durch die in Anspruch 4 angegebene Ausgestaltung wird eine zeitlich weitgehend lineare Entladung des Integrators erzielt. Nach Anspruch 5 ist der Integrator als an sich bekannter Umkehrintegrator mit einem Operationsverstärker und einem Gegenkopplungskondensator ausgebildet. Ein Umkehrintegrator stellt dabei ein besonders einfaches Integrierglied dar, das als regelbare Gleichspannungsquelle den gestellten Anforderungen vollkommen genügt. Insbesondere ist die Ausgangsspannung des Umkehrintegrators und somit die gewünschte Reizstrom-Maximalamplitude sehr einfach einstellbar, indem der Integrator über Tastschalter mit dem positiven bzw. negativen Betriebspotential verbindbar ist (Anspruch 6). Die Einstellung kann dabei besonders bedienungsfreundlich und bequem vollzogen werden, wenn der Umkehrintegrator linear mit der Zeit ent- bzw. aufladbar ist.

Die Verwendung eines Feldeffekt-Operationsverstärkers (Anspruch 7) bringt besondere Sicherheitvorteile mit sich. Die obengenannte medizinische Geräteverordnung schreibt nämlich vor, daß bei einem Ausfall der Netzversorgung und deren Wiederkehr an den Applikationselektroden des Reizstromgerätes nur eine minimale Spannung anliegen darf. Diese Forderung wird durch eine Ausgestaltung des Erfindungsgegenstandes nach Anspruch 7 erfüllt. Der im Prinzip unendliche Innenwiderstand eines Feldeffekt-Operationsverstärkers im normalen Betrieb bricht nämlich bei Ausfall seiner Betriebsspannung zusammen, der Operationsverstärker wird zwischen seinen Eingängen und den Ausgängen leitend. Damit wird der Integrator innerhalb kürzester Zeit entladen, bei Wiedereinsetzen der Betriebsspannung liegt am Integrator und damit am Gleichspannungsregler für das Reizstromgerät keine oder nur eine sehr geringe Spannung an Die Applikationselektroden sind damit - wie gefordert - im wesentlichen spannungslos.

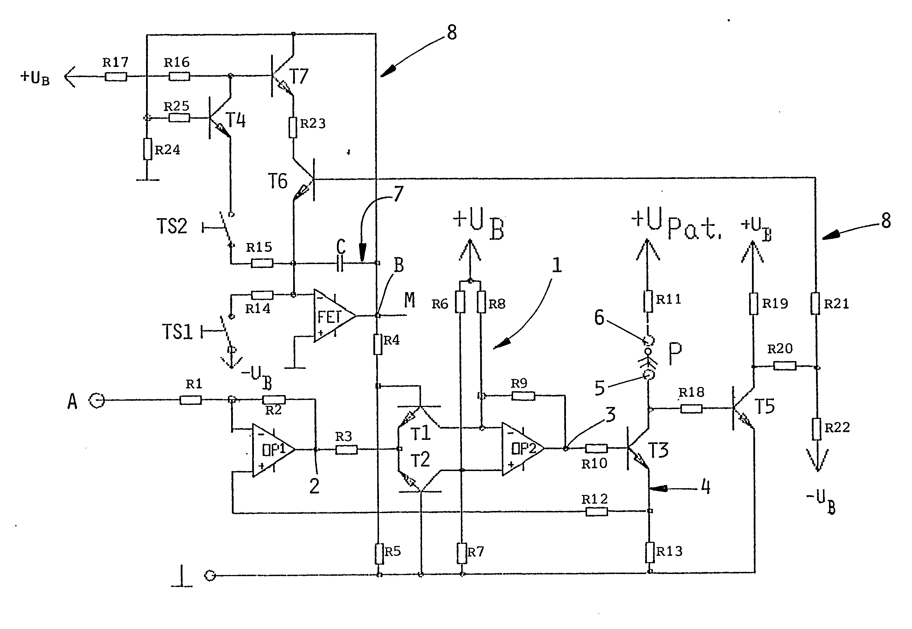

Die Erfindung wird in einem Ausführungsbeispiel anhand der beiliegenden Zeichnung näher erläutert. Letzere zeigt den Schaltplan des erfindungsgemäßen Reizstromgerätes, wobei auf den Funktionsgenerator verzichtet wurde.

Die im unteren Teil des Schaltplanes gezeigte Ansteuerschaltung zur Steuerung des Patientenstromkreises (4) weist zwei Eingänge auf, nämlich den Impulsspannungseingang (A) und den Eingang (B) für die regelbare Gleichspannung. Der Impulsspannungseingang (A) ist mit dem Ausgang eines nicht dargestellten Funktionsgenerators verbunden, der beispielsweise Dreiecks-, Sägezahn- und/oder Rechteckimpulse einstellbarer Frequenz und positiver Polarität erzeugt. Falls ein zeitlich konstanter Patientenstrom gewünscht wird, kann der Funktionsgenerator auch eine positive Gleichspannung erzeugen. Der Impulsspannungseingang (A) ist über einen Vorschaltwiderstand (R1) mit dem invertierenden Eingang des Operationsverstärkers (OP1) verbunden, dessen Ausgang über den Rückkoppelwiderstand (R2) auf den invertierenden Eingang rückgekoppelt ist.

Die beiden Transistoren (T1, T2) und der Eingangswider stand (R3), die Spannungsteiler (R4,R5 bzw. R6,R7), der Widerstand (R8) sowie der über den Rückkoppelwiderstand (R9) vom Ausgang auf den invertierenden Eingang rückgekoppelte Operationsverstärker (OP2) bilden den in seinem Aufbau und seiner Wirkungsweise wohlbekannten Zwei-Quadranten-Steilheits-Multiplizierer (1) mit seinen beiden Eingängen (2,B). Am Eingang (2) liegen die invertierten, negativen Spannungsimpulse des Funktionsgenerators an, wogegen am Eingang (B) die regelbare Gleichspannung zur Einstellung der Reizstrom-Maximalamplitude anliegt. Durch die Multiplikationsfunktion des Zwei-Quadranten-Multiplizierers (1) entsteht an dessen Ausgang (3) eine Spannung, die den Spannungsimpulsen des Funktionsgenerators kurvengetreu folgt, wobei die Maximalamplitude proportional zu der am Eingang (B) anliegenden Gleichspannung ist. Der Ausgang (3) steuert über den Basiswiderstand (R10) den Leistungstransistor (T3) im Patientenstromkreis (4). Letzterer besteht aus in Serie geschaltet dem Widerstand (R11), dem Patienten (P), dessen zu behandelnde Körperpartie über Elektroden (5,6) an den Stromkreis (4) angeschlossen ist, der Kollektor-Emitterstrecke des Leistungstransistors (T3) und dem Widerstand (R13). Die Versorgungsspannung ($U_{pat}$) von etwa 160 V für den Patientenstrom fällt über die vorgenannten Bauteile gegen Masse ab.

Durch die Stromeinprägung über den Leistungstransistor (T3) folgt der dem Patientenstrom entsprechende Kollektor-Emitterstrom des Leistungstranistors (T3) streng dessen Basis-Emitterstrom. Damit fließt bei konstanten thermischen Verhältnissen am Leistungstransistor (T3) im Patientenstromkreis (4) ein Patientenstrom, dessen Maximalamplitude unabhängig vom Patientenwiderstand ist und der dem durch die Spannungsimpulse vom Funktionsgenerator und die regelbare Gleichspannung gegebenen Sollverlauf folgt. Um Abweichungen davon aufgrund thermischer Änderungen im Leistungstransistor (T3) auszugleichen, wird die am Widerstand (R13) abfallende, dem jeweiligen Patientenstrom proportionale Spannung über den Rückkoppelwiderstand (R12) auf den nicht invertierenden Eingang des Operationsverstärkers (OP1) gelegt.

Die am Eingang (B) anliegende Gleichspannung wird durch den Integrator (7) erzeugt, der aus dem Feldeffekt-Operationsverstärker (FET) und dem dessen Ausgang (entspricht dem Eingang B für die regelbare Gleichspannung) auf den invertierenden Eingang rückkoppelnden Gegenkopplungskondensator (C) besteht. Der Integrator (7) ist durch eine Verbindung des invertierenden Einganges des Feldeffekt-Operationsverstärkers (FET) über einen Vorschaltwiderstand (R14) und den Tastschalter (TS1) mit der negativen Betriebsspannung (-$U_B$) verbindbar. Durch Schließen des Tastschalters (TS1) kann die Ausgangsspannung des Integrators (7) mit der durch den Kehrwert des Produktes aus der Kapazität des Gegenkopplungskondensators und dem Wert des Vorschaltwiderstandes (R14) gegebenen Zeitkonstante hochgefahren werden.

Weiterhin ist der invertierende Eingang des Feldeffekt-Operationsverstärkers (FET) über einen Vorschaltwiderstand (R15), einen zweiten Tastschalter (TS2), die Kollektor-Emitterstrecke des Transistors (T4) und zwei weitere Widerstände (R16,R17) mit der positiven Betriebsspannung ($U_B$) verbindbar. Durch Schließen des Tastschalters (TS2) kann der Integrator (7) mit der entsprechenden Zeitkonstanten entladen und damit dessen Ausgangsspannung erniedrigt werden.

Der Eingang (B) des Multiplizierers (1) bzw. der Ausgang des Integrators (7) dient als Meßpunkt (M) für die Gleichspannung, die proportional zur Maximalampli tude des fließenden Patientenstromes ist. Durch eine einfache Eichung des nicht dargestellten Spannungsmeßinstrumentes in Einheiten der Stromamplitude ist also eine einfache Ablesung des maximalen Patientenstromes ohne Verwendung aufwendiger Spitzenstrommeßgeräte möglich.

Wie eingangs erwähnt, können sich Probleme ergeben, wenn der zwischen den beiden Elektroden (5,6) herrschende Widerstand bzw. Impedanz solche hohen Werte annimmt, daß der tatsächlich erreichbare, maximale Patientenstrom - der durch den Quotienten aus der maximalen Versorgungsspannung ($U_{pat}$) für den Patientenstromkreis (4) und dessen Gesamtwiderstand gegeben ist - geringer ist, als die durch die Höhe der Gleichspannung am Eingang (B) gegebene Reizstrom-Maximalamplitude. Dann ist die gesamte Schaltung übersteuert und die am Meßpunkt (M) ermittelte Maximalamplitude des Reizstromes entspricht nicht dem tatsächlichen Wert. Falls die Elektroden (5,6) darüber hinaus beispielsweise vom Patienten abfallen, so stünde im ungünstigsten Falle die volle Versorgungsspannung ($U_{pat}$) an den offenen Elektroden an. Bei Wiederanlegen würde der Patient (P) einen nicht unerheblichen Stromschlag erleiden. Um die vorgenannten Störfälle und Gefahren auszuschalten, weist das erfindungsgemäße Reizstromgerät zusätzlich die Impedanz-Erfassungsschaltung (8) auf. Diese ist zwischen den Patientenstromkreis (4) und den Integrator (7) geschaltet und begrenzt dessen Spannung auf den Bereich regelbarer Stromkonstanz, was für den Fall abgelöster Elektroden bedeutet, daß diese im wesentlichen spannungslos gemacht werden. Die Impedanzerfassungsschaltung (8) ist mit der versorgungsspannungsabseitigen Elektrode (5) verbunden und weist als erstes einen Triggertransistor (T5) auf, der basisseitig über einen Basiswiderstand (R18) mit der Elektrode (5) verbunden ist. Der Emitter des Trigger transistors (T5) liegt an Masse. Sein Kollektor ist zum einen über den Widerstand (19) mit der positiven Betriebsspannung ($U_B$) verbunden, zum anderen ist der Kollektor über einen weiteren Widerstand (R20) mit dem Mittenabgriff eines Spannungsteilers (R21,R22) verbunden. Auf seiner einen Seite liegt dieser Spannungsteiler (R21,R22) an der negativen Betriebsspannung (-U$_B$) an, auf der anderen Seite ist dieser mit der Basis eines Schalttransistors (T6) verbunden. Der Emitteranschluß dieses Schalttransistors (T6) ist mit dem invertierenden Eingang des Feldeffekt-Operationsverstärkers (FET) gekoppelt. In Reihe mit seiner Kollektor-Emitterstrecke liegt ein weiterer Widerstand (R23) und die Kollektor-Emitter-Strecke eines weiteren Transistors (T7). Dessen Emitter ist über einen Widerstand (R24) mit dem Masseanschluß sowie mit dem Eingang (B) für die regelbare Gleichspannung verbunden. Die Basis des Transistors (T7) ist über die Widerstände (R16 und R 17) mit der positiven Betriebsspannung (U$_B$) verbunden.

Die vorstehend beschriebene Impedanz-Erfassungsschaltung (8) arbeitet wie folgt:

Bei ordnungsgemäß angelegten Elektroden (5,6) und einer Patientenimpedanz im Normalbereich liegt die Elektrode (5) auf einem solchen Potential, daß der Triggertransistor (T5) leitend ist. Dies bedeutet, daß am Mittenabgriff des Spannungsteilers (R21,R22) kein Schaltsignal anliegt. Sobald die Impedanz zwischen den Elektroden (5,6) in solche Bereiche steigt, daß der Bereich der riegelbaren Stromkonstanz verlassen wird - die Schaltung also übersteuert ist - sperrt der Triggertransistor (T5) und am Mittenabgriff des Spannungsteilers (R21,R22) steht ein Schaltsignal an. Dieses öffnet den Schalttransistor (T6), wodurch der Integrator (7) mit einer durch den Widerstand (R23) und die Kapazität des Gegenkopplungskondensators (C) gegebenen Zeitkonstante entladen wird. Die für die Reizstrom-Maximalamplitude repräsentative Eingangsspannung am Zwei-Quadranten-Steilheits-Mutiplizierer (1) sinkt also, bis der Spannungsabfall zwischen den beiden Elektroden (5,6) so gering ist, daß der Triggertransistor (T5) wieder leitend wird und das Schaltsignal für den Schalttransistor (T6) abfällt. Letzterer sperrt wieder, die Spannung des Integrators (7) ist also auf einen Bereich zurückgeführt, in dem regelbare Stromkonstanz gegeben ist. Falls eine oder beide Elektroden (5,6) vom Patienten abgefallen sind, bleibt der Schalttransistor (T6) gesteuert über den Triggertransistor (T5) so lange offen, bis der Integrator (7) völlig entladen ist. An den Elektroden liegt dann keine oder nur eine minimale Spannung an, wodurch diese dem Patienten wiederum gefahrlos angelegt werden können. Zur neuerlichen Einstellung der gewünschten Reizstrom-Maximalamplitude genügt es, den Tastschalter (TS1) zu schließen, bis die der gewünschten ReizstromMaximalamplitude entsprechende Gleichspannung am Integrator durch Aufladung erreicht ist. Es ist darauf hinzuweisen, daß der Transistor (T7) in Verbindung mit dem Widerstand (R23) dazu dient, eine mit der Zeit lineare Entladung des Integrators (7) bei leitendem Schalttransistor (T6) zu erzielen.

Durch die Verwendung eines Feldeffekt-Operationsverstärkers (FET) im Integrator (7) ist gewährleistet, daß letzterer bei Ausfall der Betriebsspannung (beispielsweise bei einem Netzausfall) vollkommen entladen wird. Bei wiederkehrender Netzspannung und damit der Betriebsspannung des Integrators (7) ist die Gleichspannung am Eingang (B) gleich Null oder minimal, wodurch die Elektroden (5,6) spannungslos sind.

Bezugszeichen

1 Zwei-Quadranten-Steilheitsmultiplizierer
2 Eingang
3 Ausgang
4 Patientenstromkreis
5,6 Elektroden
7 Integrator
8 Impedanzerfassungsschaltung
A Impulsspannungseingang
B Eingang für regelbare Gleichspannung
M Meßpunkt
P Patient
U$_B$ Betriebsspannung
U$_{pat}$ Versorgungsspannung für Patientenstrom
R1 Vorschaltwiderstand
R2 Rückkoppelwiderstand
R3 Eingangswiderstand
R4 Spannungsteiler
R5 Spannungsteiler
R6 Spannungsteiler
R7 Spannungsteiler
R8 Widerstand
R9 Rückkoppelwiderstand
R10 Basiswiderstand
R11 Widerstand
R12 Rückkoppelwiderstand
R13 Widerstand
R14 Vorschaltwiderstand
R15 Vorschaltwiderstand
R16 Widerstand
R17 Widerstand
R18 Basiswiderstand
R19 Widerstand
R20 Widerstand
R21 Spannungsteiler
R22 Spannungsteiler
R23 Widerstand
R24 Widerstand
R25 Widerstand
OP1 Operationsverstärker
OP2 Operationsverstärker

T1 Transistor
T2 Transistor
T3 Leistungstransistor
T4 Transistor
T5 Triggertransistor
T6 Schalttransistor
T7 Transistor
FET Feldeffekt-Operationsverstärker
C Gegenkopplungskondensator
TS1 Tastschalter
TS2 Tastschalter


**Ansprüche**

1. Reizstromgerät zur Elektrotherapie und/oder elektromedizinisch-diagnostischen Anwendung unter Applikation eines Patientenstromes vorwählbarer Kurvenform und einstellbarer Maximalamplitude mit
- einem Funktionsgenerator zur Erzeugung von Spannungsimpulsen vorwählbarer Kurvenform,
- einer regelbaren Gleichspannungsquelle zur Einstellung der Reizstrom-Maximalamplitude,
- einer dem Funktionsgenerator und der Gleichspannungsquelle nachgeschalteten Ansteuerschaltung zur Steuerung des Patientenstromkreises (4) entsprechend der vorgewählten Kurvenform und eingestellten Reizstrom-Maximalamplitude und
- einer Überwachungsschaltung zur Überwachung der zwischen den Applikationselektroden (5,6) des Patientenstromkreises (4) auftretenden Impedanz,
dadurch gekennzeichnet,
daß die regelbare Gleichspannungsquelle als auf- und entladbarer Integrator (7) ausgebildet ist, der in seiner Spannung mittels einer zwischen ihm und dem Patientenanschluß (Elektroden 5,6) des Patientenstromkreises (4) eingeschalteten Impedanzerfassungsschaltung (8) auf den Bereich regelbarer Stromkonstanz begrenzbar ist.

2. Reizstromgerät nach Anspruch 1,
dadurch gekennzeichnet,
daß die Impedanzerfassungsschaltung einen basisseitig mit dem versorgungsspannungs-abseitigen Patientenanschluß (Elektrode 5) verbundenen Triggertransistor (T5) enthält, an dessen Kollektor ein Steuersignal entsprechend der jeweils zwischen den Applikationselektroden (5,6) herrschenden Impedanz erzeugbar ist.

3. Reizstromgerät nach Anspruch 2,
dadurch gekennzeichnet,
daß mit dem Steuersignal ein weiterer Transistor (Schalttransistor T6) der Impedanzerfassungsschaltung (8) schaltbar ist, über dessen Kollektor-Emitterstrecke der Integrator (7) zur Spannungsbegrenzung auf den Bereich regelbarer Stromkonstanz entladbar ist.

4. Reizstromgerät nach Anspruch 3,
dadurch gekennzeichnet,
daß der Kollektor-Emitter-Strecke des vorgenannten Transistors (Schalttransistor T6) die Kollektor-Emitter-Strecke eines weiteren Transistors (T7) vorgeschaltet ist.

5. Reizstromgerät nach einem der vorgenannten Ansprüche,
dadurch gekennzeichnet,
daß der Integrator (7) als an sich bekannter Umkehrintegrator ausgebildet ist, der einen Operationsverstärker und einen Gegenkopplungskondensator (C) zwischen seinem Ausgang und seinem invertierenden Eingang aufweist.

6. Reizstromgerät nach Anspruch 5,
dadurch gekennzeichnet,
daß der Umkehrintegrator über Tastschalter (TS1,TS2) jeweils gegen positives bzw. negatives Betriebspotential linear mit der Zeit ent- bzw. aufladbar ist.

7. Reizstromgerät nach Anspruch 5 oder 6,
dadurch gekennzeichnet,
daß der Operationsverstärker des Umkehrintegrators (7) ein Feldeffekt-Operationsverstärker (FET) ist.

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-3 447 892 (VON NETTELHORST) <br> * Seiten 1-4; Seite 10, Zeile 1 - Seite 11, Zeile 24; Figuren * <br> --- | 1,5,6 | A 61 N 1/08 <br> A 61 N 1/18 |
| A | EP-A-0 001 806 (SIEMENS) <br> * Seite 1, Zeilen 7-12; Seite 2, Zeile 22 - Seite 4, Zeile 15; Seite 4, Zeile 26 - Seite 6, Zeile 4; Figuren * <br> --- | 1-4 | |
| A | FR-A-2 500 689 (FAIVELEY) <br> * Insgesamt * <br> ----- | 1 | |

| | |
|---|---|
| | RECHERCHIERTE SACHGEBIETE (Int. Cl.4) |
| | A 61 N |

Der vorliegende Recherchenbericht wurde für alle Patentansprüche erstellt

| Recherchenort | Abschlußdatum der Recherche | Prüfer |
|---|---|---|
| DEN HAAG | 22-06-1989 | LEMERCIER D.L.L. |